(19) Europäisches Patentamt
European Patent Office
Office européen des brevets

(11) Veröffentlichungsnummer: **0 082 111**
**A2**

# (12) EUROPÄISCHE PATENTANMELDUNG

(21) Anmeldenummer: **82810497.6**

(22) Anmeldetag: **22.11.82**

(51) Int. Cl.³: **C 07 D 233/54**, C 07 D 401/04

(30) Priorität: **27.11.81 CH 7611/81**

(43) Veröffentlichungstag der Anmeldung: **22.06.83**
**Patentblatt 83/25**

(84) Benannte Vertragsstaaten: **AT BE CH DE FR GB IT LI LU NL SE**

(71) Anmelder: **CIBA-GEIGY AG, Patentabteilung Postfach, CH-4002 Basel (CH)**

(72) Erfinder: **Aufderhaar, Ernst, Dr., Ziegelhofweg 26, CH-4303 Kaiseraugst (CH)**
Erfinder: **Meier, Andres, Mühleweg 5, CH-4105 Biel-Benken (CH)**

## (54) Verfahren zur Herstellung von Imidazolen.

(57) Neuartiges Verfahren zur Herstellung von Imidazolen der Formel

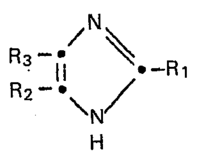

(I)

worin $R_1$ sowie mindestens einer der Reste $R_2$ und $R_3$ einen über ein Kohlenstoffatom gebundenen organischen Rest und ein davon verschiedener Rest $R_2$ oder $R_3$ Wasserstoff darstellt, wobei über gesättigte Kohlenstoffatome gebundene nichtaromatische Reste $R_2$ und $R_3$ auch miteinander verbunden sein können, mit der Massgabe, dass $R_1$ von Resten der Formeln $-A-O-R_4$, $-A-S(O)_n-R_4$ und $-A-R_5$, in denen A einen zweiwertigen Kohlenwasserstoffrest, $R_4$ einen gegebenenfalls substituierten Kohlenwasserstoffrest, n 0, 1 oder 2 und $R_5$ gegebenenfalls verestertes oder amidiertes Carboxy bedeutet, verschieden ist, wenn $R_2$ und $R_3$ aromatische Reste darstellen, und ihrer Salze, dadurch gekennzeichnet, dass man ein reaktionsfähiges verestertes Hydroxyketon der Formel

$$R_2\text{-}CH(X)\text{-}C(=O)\text{-}R_3 \qquad (IX)$$

worin X eine reaktionsfähige versterte Hydroxygruppe darstellt, oder ein Salz davon, mit einem Ammoniumsalz der Formel

$$R_1\text{-}COO^{\ominus}NH_4^{\oplus} \qquad (X)$$

erforderlichenfalls in Gegenwart von Ammoniak oder eines Ammoniakliefernden Mittels, umsetzt.

EP 0 082 111 A2

CIBA-GEIGY AG
Basel (Schweiz)

4-13685/-

Verfahren zur Herstellung von Imidazolen

Gegenstand der Erfindung ist ein neuartiges Verfahren zur Herstellung von Imidazolen der Formel

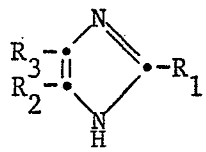

(I),

worin $R_1$ sowie mindestens einer der Reste $R_2$ und $R_3$ einen über ein Kohlenstoffatom gebundenen organischen Rest und ein davon verschiedener Rest $R_2$ oder $R_3$ Wasserstoff darstellt, wobei über gesättigte Kohlenstoffatome gebundene nicht-aromatische Reste $R_2$ und $R_3$ auch miteinander verbunden sein können, mit der Massgabe, dass $R_1$ von Resten der Formeln $-A-O-R_4$, $-A-S(O)_n-R_4$ und $-A-R_5$, in denen A einen zweiwertigen Kohlenwasserstoffrest, $R_4$ einen gegebenenfalls substituierten Kohlenwasserstoffrest, n 0, 1 oder 2 und $R_5$ gegebenenfalls verestertes oder amidiertes Carboxy bedeutet, verschieden ist, wenn $R_2$ und $R_3$ aromatische Reste darstellen, und ihrer Salze.

Imidazole der Formel I haben vielfältige gewerbliche Anwendungsmöglichkeiten. So werden Verbindungen der Formel I, worin mindestens zwei der Reste $R_1$, $R_2$ und $R_3$ unabhängig voneinander Furyl, Thienyl, Pyridyl, Phenyl, Niederalkylphenyl, Niederalkoxyphenyl, Niederalkylthiophenyl, Halogenphenyl, Trifluormethylphenyl, 3,4-Dimethoxyphenyl bzw. 2,4-Dichlorphenyl bedeuten und ein davon verschiedener Rest Trifluormethyl oder Alkyl bedeutet, und ihre Salze in der US-Patentschrift Nr. 3,707,475 als Antiinflammatorika vorgeschlagen. Ferner ist aus J. Chem. Soc., 278 (1941) bekannt, dass 2-substituierte 4,5-Diphenylimidazole, deren 2-Substituent Aryl oder Alkyl ist, Chemieluminiszens-Eigenschaften haben und aus der DE-Patentschrift 1,106,599, dass 2,4,5-Triarylimidazole phototrope, elektrophotographische und xerographische Verwendungsmöglichkeit aufweisen.

Ferner weisen Imidazole der Formel I, worin $R_1$ Niederalkyl, Cycloalkyl oder gegebenenfalls durch Niederalkyl, Niederalkoxy oder Halogen substituiertes Phenyl ist und einer der Reste $R_2$ und $R_3$ gegebenenfalls durch Niederalkyl, Niederalkoxy, Niederalkylthio, Niederalkansulfonyl, Hydroxy oder Halogen substituiertes Phenyl und der andere einen gegebenenfalls N-oxidierten 6-gliedrigen heteroaromatischen Rest mit 1 oder 2 Stickstoffatomen bedeutet, gemäss der US-Patentschrift 3,929,807 antiinflammatorische Eigenschaften auf.

Die Erfindung betrifft dementsprechend ebenso die Anwendung des neuartigen Verfahren auf die Herstellung bekannter Imidazole der Formel I, beispielsweise solcher, worin mindestens zwei der Reste $R_1$, $R_2$ und $R_3$ unabhängig voneinander Furyl, Thienyl, Pyridyl, Phenyl, Niederalkylphenyl, Niederalkoxyphenyl, Niederalkylthiophenyl, Halogenphenyl, Trifluormethylphenyl, 3,4-Dimethoxyphenyl bzw. 2,4-Dichlorphenyl bedeuten und ein davon verschiedener Rest Trifluormethyl oder Alkyl bedeutet, oder worin $R_1$ Niederalkyl, Cycloalkyl oder gegebenenfalls durch Niederalkyl, Niederalkoxy oder Halogen substituiertes Phenyl ist und einer der Reste $R_2$ und $R_3$ gegebenenfalls durch Niederalkyl, Niederalkoxy, Niederalkylthio, Niederalkansulfonyl, Hydroxy oder Halogen substituiertes Phenyl und der andere einen gegebenenfalls N-oxidierten 6-gliedrigen heteroaromatischen Rest mit 1 oder 2 Stickstoffatomen bedeutet.

Es sind zwar bereits einige Verfahren zur Herstellung von Imidazolen der Formel I bekannt, die aber sämtlich prinzipielle Nachteile aufweisen, die ihre Anwendungsmöglichkeiten einschränken bzw. ihrer Uebertragung im industriellen Massstab im Wege stehen.

So werden Imidazole der Formel I üblicherweise hergestellt, indem man ein Diketon der Formel $R_2-C(=O)-C(=O)-R_3$ (II) mit Ammoniak oder einem Ammoniak abgebenden Mittel und einem Aldehyd der Formel $R_1-CHO$ (III) umsetzt. Dieses Verfahren, das beispielsweise in der CH-Patentschrift 561,202 eingehend beschrieben ist, hat den Nachteil, dass die Ausgangsstoffe der Formeln II und III in ungünstiger Oxidationsstufe verwendet werden. So müssen die Diketone der Formel III im allgemeinen zunächst durch einen vorgelagerten Oxidationsschritt unter Bildung der 1,2-Dioxoäthylengruppe aus der 1- bzw. 2-Oxoäthylengruppe, 1-Hydroxy-2-oxo- bzw. 2-Hydroxy-1-oxo-äthylengruppe oder Vinylgruppe aus leichter zugänglichen Primärprodukten hergestellt werden. So werden die Diketone der Formel III gemäss der schweizerischen Patentschrift Nr. 561,202 beispielsweise durch Oxidation entsprechender Monoketone der Formel $R_2-CH_2-C(=O)-R_3$ (IV) bzw. Styrylverbindungen der Formel $R_2-CH=CH-R_3$ (V) mittels Selendioxid oder durch Oxidation entsprechender Hydroxyketone der Formel $R_2-CH(OH)-C(=O)-R_3$ (VI) mittels Kupfer-II-salzen erhalten. Die hohe Toxizität des im ersten Falle verwendeten Selendioxides und der entstehenden Reduktionsprodukte stellt ein grosses Sicherheits- und Umweltrisiko dar, das sich, wenn überhaupt nur mit grossem Aufwand in vertretbaren Grenzen halten lässt. Im zweiten Falle werden die Reaktionsprodukte der Formel I in Form der Kupfer-IIkomplexe erhalten. Aus diesen müssen die gewünschten Imidazole freigesetzt werden. Hierzu wird die Umsetzung mit physiologisch bedenklichem Schwefelwasserstoff ausgeführt. Ein weiterer Nachteil besteht darin, dass das als weiteres Reaktionsprodukt anfallende Kupfersulfid, welches in erheblicher Weise die Umwelt belastet, entweder vernichtet, endgelagert oder wieder aufgearbeitet werden muss. Ebenso stehen die Aldehyde der Formel (IV) nur selten in grösseren Mengen zur Verfügung. Sie müssen vielmehr erst ausgehend von den entsprechenden Carbonsäuren bzw. Carbonsäurehalogeniden durch Reduktion gebildet werden.

In einer Variation zu dem vorstehenden Verfahren gibt die schweizerische Patentschrift Nr. 561,202 an, dass man Verbindungen der Formel V zunächst mittels salpetriger Säure α-oximieren und das gebildete Oxim der Formel $R_2-C(=NOH)-C(=O)-R_3$ (VIII) einsetzen kann. Dabei werden jedoch 1-N-Oxide von Verbindungen der Formel I erhalten, aus denen diese durch nachfolgende Reduktion freigesetzt werden müssen.

Ferner ist bekannt und beispielsweise in der CH-Patentschrift 561 716 beschrieben, N-acylierte α-Aminoketone der Formel $R_2-CH(NHCOR_1)-C(=O)-R_2$ (VIII) mit Ammoniak oder Ammoniak freisetzenden Mitteln umzusetzen. Dieser Verfahrensweg hat den Nachteil, dass die Aminoketone nur über mehrere, im technischen Massstab aufwendige Syntheseschritte erhalten werden können. Beispielsweise gelangt man zu den Aminoketonen, indem man entsprechende Hydroxyketone in Oxime überführt und diese anschliessend mit Wasserstoff zu den Aminoketonen reduziert, die zudem bekanntermassen wenig stabil sind. Die N-Acylierung erfolgt schliesslich durch Umsetzung mit reaktionsfähigen Säurederivaten, welche wiederum erst aus Carbonsäuren hergestellt werden müssen.

Bei einem weiteren Verfahren, beschrieben z.B. in der CH-Patentschrift 561,717, wird ein reaktionsfähig verestertes α-Hydroxyketon der Formel $R_2-CH(X)-C(=O)-R_3$ (IX), worin X reaktionsfähiges verestertes Hydroxy, z.B. Halogen, insbesondere Chlor oder Bromid bedeutet, mit einem Amidin der Formel $R_1-C(=NH)-NH_2$ (XII) kondensiert. Dadurch wird zwar der vorstehend erwähnte Umweg über Aminoketone vermieden, jedoch erfordert die Herstellung der Amidine, welche zudem nur in Salzform stabil sind und eingesetzt werden können, ebenfalls einen mehrstufigen Verfahrensweg, wobei man üblicherweise von ökologisch bedenklichen Nitrilen ausgeht, die wiederum aus anderen Carbonsäure-Derivaten hergestellt werden müssen. In einer Abwandlung dieses Verfahrens kann man gemäss Chem. Ber. 86, 88-93 das Hydroxy- bzw. Halogenketon mit Formamid umsetzen und so zu 2-unsubstituierten Imidazolen gelangen. Das Verfahren lässt sich

jedoch, wie in loc. cit. ausdrücklich ausgeführt, nicht auf andere Carbonsäureamide übertragen und kommt somit als Herstellungsverfahren der 2-substituierten Imidazole der Formel I prinzipiell nicht in Betracht.

Eine weitere Synthesemöglichkeit für Imidazole der Formel I kann man der schweizerischen Patentschrift Nr. 561 718 entnehmen, die die Herstellung ausgehend von Oxazolen und Ammoniak und/oder Formamid beschreibt. Nachteilig ist auch hier der zusätzlich erforderliche mehrstufige Syntheseweg für die Herstellung der Oxazolzwischenprodukte.

So setzt man beispielsweise Hydroxyketone der Formel VII mit reaktiven Derivaten von Carbonsäuren der Formel $R_1$-COOH (X) zu den entsprechenden Estern um, behandelt diese anschliessend mit einem Ammoniak freisetzenden Mittel und erhält so die verfahrensgemäss einzusetzenden Oxazole.

Der Erfindung lag dementsprechend die Aufgabe zugrunde, ein Verfahren zur Herstellung von Imidazol-Verbindungen der Formel I zu finden, welches sich durch ein Minimum an Umweltbelastung, durch Verwendung kostengünstiger Ausgangsstoffe, durch eine geringe Zahl an Verfahrensschritten und durch leicht handhabbare Verfahrensführung auszeichnet. Die Aufgabe wird erfindungsgemäss dadurch gelöst, dass man ein reaktionsfähiges verestertes Hydroxyketon der Formel $R_2$-CH(X)-C(=O)-$R_3$ (IX), worin X eine reaktionsfähige veresterte Hydroxygruppe darstellt, oder ein Salz davon, mit einem Ammoniumsalz der Formel

$$R_1\text{-COO}^{\ominus}\ NH_4^{\oplus} \qquad (X),$$

umsetzt, erforderlichenfalls in Gegenwart von Ammoniak oder eines Ammoniak-liefernden Mittels.

Als Ammoniak-liefernde Mittel kommen dabei neben einem Ueberschuss des Salzes der Formel X beispielsweise Ammoniumsalze einer verglichen mit der Säure der Formel $R_1$-COOH (XI) schwächeren Säure, wie Ammoniumcarbonat bzw. -carbaminat in Betracht.

Die Erfindung betrifft demnach gleichermassen diejenigen Verfahrensvarianten, wonach man eine Verbindung der Formel IX mit der mindestens doppeltmolaren Menge eines Ammoniumsalzes der Formel X oder eine Verbindung der Formel IX mit der etwa äquimolaren Menge eines Ammoniumsalzes der Formel X und der mindestens äquimolekularen Menge, vorzugsweise jedoch der etwa 3- bis 5-fach molaren Menge von Ammoniak oder eines Ammoniak-liefernden Mittels umsetzt.

Eine reaktionsfähige veresterte Hydroxygruppe ist beispielsweise eine vorzugsweise durch starke anorganische oder organisch Säuren, wie starke Mineralsäuren, z.B. Halogenwasserstoffsäuren, wie Chlor- oder Bromwasserstoffsäure, oder starke organische Sulfonsäuren, wie entsprechende Niederalkan- oder Arylsulfonsäuren, z.B. Methan- oder eine gegebenenfalls substituierte Benzolsulfonsäure, veresterte Hydroxygruppe und stellt z.B. Halogen, wie Chlor oder Brom, Niederalkylsulfonyloxy, z.B. Methyl- oder Ethylsulfonyloxy, oder Arylsulfonyloxy, z.B. p-Toluol- oder Benzolsulfonyloxy, dar.

Das Ammoniumsalz der Formel X kann auch unter den Reaktionsbedingungen _in situ_ gebildet werden, beispielsweise indem man im Reaktionsgemisch die Säure der Formel XI vorlegt und mit flüssigem oder gasförmigem Ammoniak versetzt. Bei dieser Ausführungsform kann das Ammoniak auch in Form eines Salzes mit einer verglichen mit der Säure der Formel XI schwächeren Säure, wie eines Salzes der Kohlensäure, z.B. als Ammoniumcarbonat oder -carbaminat, zugegeben werden.

Die Reaktion wird in An- oder Abwesenheit eines unter den Reaktionsbedingungen inerten Lösungsmittels bei erhöhter Temperatur durchgeführt, beispielsweise bei 60° bis 180°C, vorzugsweise in einem Temperaturbereich von 90° bis 120°C.

Als geeignete Lösungsmittel kommen z.B. gegebenenfalls halogenierte Kohlenwasserstoffe, wie gegebenenfalls halogenierte aliphatische, cycloaliphatische oder aromatische Kohlenwasserstoffe, wie Hexan, Cyclohexan, Toluol, Chloroform, oder Chlorbenzol, Alkanole, wie Propanol, Isopropanol, Butanole, Pentanole oder Octanole, Ether, wie Dimethoxyethan, Ethylenglykolmonoethylether, Dioxan oder Tetrahydrofuran, Niederalkancarbonsäuren, wie Ameisen- oder Essigsäure oder vorzugsweise Säuren der Formel XI, Amide, wie Niederalkancarbonsäureamide, z.B. Formamid oder Dimethylformamid, oder Lactame, z.B. N-Methylpyrrolidon, Sulfoxide, wie Dimethylsulfoxid, oder Wasser in Frage.

Eine bevorzugte Ausführungsform der erfindungsgemässen Herstellung von Verbindungen der Formel I besteht darin, dass man eine Verbindung der Formel IX worin X beispielsweise Halogen, wie Brom, bedeutet, bei einer Reaktionstemperatur von etwa 100°C mit einem Ammoniumsalz der Formel X umsetzt. Die Verbindung der Formel X wird dabei im Ueberschuss zugegeben, z.B. in einem Verhältnis gegenüber dem Ester der Formel IX von etwa 4:1 bis etwa 6:1, und lässt sich <u>in situ</u> bilden, indem man z.B. die entsprechende Säure unter den Reaktionsbedingungen mit flüssigem Ammoniak umsetzt.

In einer weiteren bevorzugten Ausführungsform des erfindungsgemässen Verfahrens kann die Säure der Formel XI im Ueberschuss zugesetzt werden und gleichzeitig als Lösungsmittel für die Reaktion dienen.

Die Ausgangsstoffe der Formel IX sind nur zum Teil bekannt.

Sie lassen sich beispielsweise durch Esterkondensation von veresterten Säuren der Formel $R_2$-$CH_2$-COOH mit veresterten Säuren der Formeln $R_3$-COOH, vorzugsweise in Gegenwart einer Base erhalten. Das resultierende $\alpha$-Methylenketon der Formel $R_2$-$CH_2$-C(=O)-$R_3$ (IV) wird

beispielsweise bromiert und somit in eine Verbindung der Formel IX überführt, worin X Brom bedeutet.

Das vorliegende erfindungsgemässe Verfahren zeichnet sich gegenüber den bekannten beschriebenen Verfahren dadurch aus, dass man ausgehend von leicht zugänglichen Ausgangsstoffen mehrere Verfahrensschritte einspart und dass keine oder in geringerem Masse ökologisch bedenkliche Abfallstoffe anfallen. Somit ist es in Ueberwindung eines in der Fachwelt bestehenden Vorurteils (Chem. Ber. loc. cit.), dass die Umsetzung von α-Halogenketonen mit höheren Säureamiden nicht zu Imidazolverbindungen führe, in überraschender Weise gelungen, ein einfaches kostengünstiges, ökologisch unbedenkliches und leicht in technischen Massstab übertragbares Verfahren für die Herstellung von Imidazolen der Formel I aus entsprechenden reaktionsfähigen veresterten α-Hydroxyketonen und Ammoniumsalzen entsprechender organischer Carbonsäuren zu entwickeln.

Die Erfindung betrifft vor allem ein Verfahren zur Herstellung von Imidazolderivaten der allgemeinen Formel I, worin $R_1$ einen verzweigten Niederalkylrest bis und mit 4 C-Atomen oder einen gegebenenfalls durch Halogen bis und mit Atomnummer 35, wie Chlor, substituierten Phenylrest darstellt und mindestens einer der Reste $R_2$ und $R_3$ einen durch Halogen bis und mit Atomnummer 35, Niederalkyl bis und mit 4 C-Atomen, Niederalkoxy bis und mit 4 C-Atomen oder Niederalkylthio mit bis und mit 4 C-Atomen, wie Methylthio, p-substituierten, durch Niederalkoxy mit bis und mit 4 C-Atomen, wie Methoxy, m- und p-substituierten oder durch Halogen der Atomnummer bis und mit 35, wie Chlor o- und p-substituierten Phenylrest und ein davon verschiedener Rest $R_2$ oder $R_3$ Pyridin bzw. 1-Oxido-pyridyl, wie 3-Pyridyl, Furyl, wie 2-Furyl, oder Thienyl, wie 2-Thienyl, bedeutet, sowie ihrer Salze.

Die Erfindung betrifft insbesondere die in den Beispielen aufgeführten Verfahren.

Die Erfindung betrifft auch diejenigen Ausführungsformen des Verfahrens, nach denen man von einer auf irgendeiner Stufe des Verfahrens erhältlichen Verbindung ausgeht und die fehlenden Verfahrensschritte durchführt, oder das Verfahren auf irgendeiner Stufe abbricht, oder bei denen man unter den Reaktionsbedingungen einen Ausgangsstoff, gegebenenfalls <u>in situ,</u> bildet.

In den nachfolgenden Beispielen werden Temperaturen in Celsiusgraden angegeben.

<u>Beispiel 1</u>: 3,57 g α-Brom-benzyl-3-pyridyl-keton-hydrobromid werden mit 5,95 g Ammoniumpivalat in 50 g Pivalinsäure 5 Stunden bei 100°C gerührt. Nach Erkalten wird die Reaktionslösung mit 50 ml Ethylacetat versetzt und mit 80 ml 25%igem Ammoniak alkalisch gestellt. Die organische Phase wird abgetrennt und die Wasserphase nochmals mit Ethylacetat extrahiert. Nach Waschen mit Wasser und Trocknen über Natriumsulfat wird das Lösungsmittel abdestilliert. Der Rückstand wird in Acetonitril heiss gelöst und durch Abkühlen zur Kristallisation gebracht. Nach Filtration und anschliessender Umlösung aus Acetonitril erhält man 2-tert.-Butyl-4(5)-phenyl-5(4)-(3-pyridyl)-imidazol, Smp. 191-192°C.

Das Ausgangsmaterial kann folgendermassen hergestellt werden :

Zu einer Lösung von 66.0 g Nikotinsäuremethylester in 52 ml Toluol werden 35.4 g Natriummethylat gegeben. Man erhitzt zum Sieden und tropft dann eine Lösung von 80.1 g Phenylessigsäuremethylester in 270 ml Toluol langsam im Lauf von 2 1/2 Stunden zu. Gleichzeitig werden am absteigenden Kühler 130 ml eines Gemischs von Methanol und Toluol abdestilliert. Man destilliert anschliessend langsam weiter, bis das Reaktionsgemisch eine Temperatur von 108° erreicht hat. Man kühlt auf Raumtemperatur ab und lässt den Ansatz unter

Kühlung zu einer Mischung von 150 ml konzentrierter Salzsäure und 130 ml Wasser fliessen. Es wird weitere 5 Stunden zum Sieden erhitzt, danach auf 10° abgekühlt und das ausgefallene Produkt abfiltriert. Es wird mit 2 x 50 ml Toluol gewaschen und im Vakuum bei 60° getrocknet. Man erhält Benzyl-3-pyridyl-keton-hydrochlorid, Smp. 219-221°, Ausbeute 99,3 g (85 % d.Th.).

Beispiel 2: 16,5 g α-Brom-benzyl-3-pyridyl-keton-hydrobromid werden mit 11,0 g Ammoniumpivalat in 70 ml Dimethylformamid auf 100°C erhitzt. Es wird 9 Stunden bei dieser Temperatur unter Einleiten von Ammoniakgas gerührt. Dann wird die Reaktionslösung im Vakuum eingedampft, der Rückstand in je 100 ml Wasser und Ethylacetat gelöst und mit 10 ml 25%igem Ammoniak auf pH 9 gestellt. Die organische Phase wird abgetrennt und die alkalische Wasserphase einmal mit Ethylacetat extrahiert. Die vereinigten organischen Phasen werden mit Wasser gewaschen und nach Trocknen über Natriumsulfat eingedampft. Der Rückstand wird heiss in Acetonitril gelöst und unter Abkühlen zur Kristallisation gebracht. Nach Filtrieren und Umlösen aus Acetonitril erhält man 2-tert.-Butyl-4(5)-phenyl-5(4)-(3-pyridyl)-imidazol, Smp. 190-191.5°C, Ausbeute 6,35 g (49,6 g d.Th.).

Das Ausgangsmaterial kann folgendermassen hergestellt werden :

Zu einer Lösung von 84.9 g Benzyl-3-pyridyl-keton in 1200 ml Aethylenchlorid werden bei 40°C innerhalb 5 Stunden 72.2 g Brom zugetropft. Anschliessend wird noch 36 Stunden bei 40°C verrührt, dann kann das α-Brom-benzyl-3-pyridyl-keton - hydrobromid durch Filtration isoliert werden (Smp. 226-230°C) , Ausbeute 134,4 g (87,6 % d. Th.).

Beispiel 3: 17,8 g α-Brom-benzyl-3-pyridyl-ketonhydrobromid und 35,75 Ammoniumpivalat, gelöst in 75 ml Dimethylformamid werden während 3 Stunden bei 100°C gerührt. Die Aufarbeitung erfolgt wie in Beispiel 2. Man erhält 2-tert.-Butyl-4(5)-phenyl-5(4)-(3-pyridyl)-imidazol vom Smp. 191-192°C, Ausbeute 8,13 g (58,7 % d.Th.).

Beispiel 4: 4,09 g Pivalinsäure werden in 25 ml Dimethylformamid gelöst. Nach Erwärmen auf 100°C werden zuerst 2,9 g Ammoniumcarbonat, dann nach einigen Minuten Rühren 2,41 g α-Brom-benzyl-3-pyridylketon-hydrobromid zugegeben. Während 9 Stunden rührt man bei 100°C unter gleichzeitiger portionenweiser Zugabe von weiteren 4,6 g Ammoniumcarbonat. Die Aufarbeitung erfolgt wie in Beispiel 2 beschrieben. Man erhält 2-tert.-Butyl-4(5)-phenyl-5(4)-(3-pyridyl)-imidazol. Smp. 190,5-192°C.

Beispiel 5: 32,85 g α-Brom-benzyl-3-pyridyl-keton-hydrobromid werden in einer Lösung von 56,4 g Pivalinsäure und 100 ml Cellosolve suspendiert. Bei Raumtemperatur wird durch Einleiten von Ammoniak die Pivalinsäure neutralisiert, dann 3 Stunden bei 100°C gerührt. Die Aufarbeitung erfolgt wie in Beispiel 2 beschrieben. Man erhält 2-tert.-Butyl-4(5)-phenyl-5(4)-(3-pyridyl)-imidazol; Smp. 190.5-191°C.

Beispiel 6: Zu 23.35 g Benzyl-3-pyridyl-keton-hydrochlorid in 210 g Pivalinsäure werden bei 85°C innerhalb 4 Stunden 17,0 g Brom zugetropft. Anschliessend wird weitere 3 Stunden bei 90°C verrührt, dann unter Vakuum soviel Pivalinsäure abdestilliert, bis nur noch die 6-fache molare Menge (bezogen auf Benzyl-3-pyridyl-keton-hydrochlorid) im Reaktionsgefäss vorliegt. Das Reaktionsgemisch, das α-Brom-Benzyl-3-pyridyl-keton-hydrobromid und Pivalinsäure enthält , wird mit 100 ml Cellosolve verdünnt. Bei 10-15°C wird nun mit Ammoniakgas die Pivalinsäure neutralisiert und das Gemisch anschliessend 3 Stunden bei 100°C verrührt. Die Aufarbeitung erfolgt wie in Beispiel 2 beschrieben. Man erhält 2-tert.-Butyl-4(5)-phenyl-5(4)-(3-pyridyl)-imidazol vom Smp. 190-190,5°C, Ausbeute 16,33 g (59,0 % d.Th.).

Beispiel 7: 23.3 g Benzyl-3-pyridyl-keton-hydrochlorid werden in 230 ml Pivalinsäure bei 85° suspendiert. Man tropft innerhalb von 6 Stunden 17 g Brom zu, vernichtet anschliessend den Bromüberschuss durch Zugabe von 10 ml Aceton und kühlt das Reaktionsgemisch auf 60° ab.

Man setzt 90 g Ammonium-pivalat zu, rührt eine halbe Stunde bei 60° und erhöht dann die Temperatur für 5 Stunden auf 100°.
Nach Abkühlen auf 40° setzt man 200 ml Essigsäureethylester und 50 ml Wasser zu, rührt bis zur vollständigen Lösung und giesst dann das Gemisch zu 200 ml 10%iger Salzsäure. Die organische Phase wird abgetrennt und zweimal mit je 50 ml 10%iger Salzsäure und einmal mit Wasser nachgewaschen.
Die vereinigten wässrigen Phasen werden auf pH 8-9 gebracht, anschliessend wird das abgeschiedene Rohprodukt in Essigsäureethylester aufgenommen und aus diesem nach Waschen mit Wasser und Eindampfen als Oel erhalten, welches durch Aufnehmen in 60 ml Acetonitril zur Kristallisation gebracht wird.
Man erhält 8.5 g (30,6 % d.Th.) 2-tert.-Butyl-4(5)-phenyl-5(4)-3-pyridyl)imidazol, Smp. 191-192°.

Das Ausgangsmaterial kann z.B. folgendermassen hergestellt werden:

21,9 g Benzyl-3-pyridyl-keton-hydrochlorid (vergl. Beispiel 1) werden in 295 ml Wasser gelöst. Nach Zugabe von 94 ml Toluol werden unter Rühren 14,2 ml Ammoniak konz. zudosiert. Nach Abtrennen der Toluolphase extrahiert man die Wasserphase noch zweimal mit je 20 ml Toluol und engt die vereinigten org. Phasen zur Trockene ein. Man erhält Benzyl-3-pyridyl-keton. Smp.: 60,2-61,5°C.

Beispiel 8: In eine Lösung von 19.73 g Benzyl-3-pyridyl-keton und 200 ml Aethylenchlorid werden bei 80°C während 13 Stunden Chlorgas eingeleitet. Das α-Chlor-Benzyl-3-pyridyl-keton-hydrochlorid kann anschliessend durch Filtration isoliert werden (Smp. 207.4-210.0°C), Ausbeute 18.3 g (68,2 % d.Th.).

Beispiel 9: 30,0 g (0,084 Mol) α-Brom-benzyl-3-pyridyl-keton-hydrobromid werden mit 60,0 g Ammoniumvalerianat in 150 ml Cellosolve auf 100°C erhitzt. Es wird 3 Std. bei dieser Temperatur verrührt, dann wird das Reaktionsgemisch eingedampft. Die Aufarbeitung erfolgt wie in

Beispiel 1. Man erhält 2-n-Butyl-4(5)-phenyl-5(4)-(3-pyridyl)-imidazol vom Smp. 117,5-119,5°C, Ausbeute 12,0 g (51,5 % d.Th.).

Beispiel 10: 18,3 g α-Chlor-benzyl-3-pyridyl-keton-hydrochlorid und 48,6 g Ammoniumpivalat werden in 120 ml Aethylenglykolmonoäthyläther suspendiert und während 3 Stunden bei 100°C verrührt. Die Aufarbeitung erfolgt wie in Beispiel 2. Man erhält 2-tert.-Butyl-4(5)-phenyl-5(4)-(3-pyridyl)-imidazol vom Smp. 190,5-191,5°C.

Beispiel 11: 27.5 g α-Brom-desoxibenzoin werden mit 42.4 g Ammoniumacetat in 100 ml Aethylenglykol-monoäthyläther 4 Stunden auf 100°C erhitzt.
Man filtriert vom Ungelösten, dampft das Filtrat im Vakuum ein und nimmt den Rückstand mit 100 ml Wasser und 100 ml Aethylacetat auf. Man stellt den pH mit wässrigem Ammoniak auf 9-10, trennt die organische Phase ab und wäscht sie mit Wasser. Nach dem Eindampfen hinterbleibt ein kristalliner Rückstand, der mit wenig kaltem Methanol aufgenommen und abfiltriert wird.
Man erhält 12.8 g (54.7 % d.Th.) 2-Methyl-4,5-diphenylimidazol, das aus Methanol umkristallisiert werden kann; Smp. 237-238°.

Beispiel 12: Analog Beispiel 11 werden 23.0 g α-Chlor-desoxibenzoin mit 42.4 g Ammoniumacetat umgesetzt.
Es werden 12.1 g (51.7 % d.Th.) 2-Methyl-4,5-diphenyl-imidazol erhalten.

Beispiel 13: In analoger Weise wie in den Beispielen 1 bis 9 beschrieben erhält man:

2-(p-Chlorphenyl)-4(5)-(p-methoxyphenyl)-5(4)-(3-pyridyl)-imidazol, Fp. 200-3°,

2-tert.-Butyl-4(5)-(p-methoxyphenyl)-5(4)-(3-pyridyl)-imidazol, Fp. 202-4°,

2-tert.-Butyl-4(5)-(o-chlorphenyl)-5(4)-(3-pyridyl)-imidazol, Fp. 205-6°,

2-tert.-Butyl-4(5)-(m-tolyl)-5(4)-(3-pyridyl)-imidazol, Fp. 170-1°,

2-tert.-Butyl-4(5)-(p-methylthiophenyl)-5(4)-(3-pyridyl)-imidazol, Fp. 199-200°,

2-tert.-Butyl-4(5)-(p-methoxyphenyl)-5(4)-(3-pyridyl-1-oxido)-imidazol, Fp. 250-8°, und

2-Trifluormethyl-4,5-bis(p-methoxyphenyl)-imidazol, Smp. 192-194°,

2-tert.-Butyl-4,5-bis-phenyl-imidazol.

Beispiel 14: In analoger Weise wie in den Beispielen 1-12 beschrieben erhält man

2-Isobutyl-4,5-bis-phenyl-imidazol, Smp. 211-214°,

2-(p-Bromphenyl)-4,5-bis-phenyl-imidazol, Smp. 262-264° und

5-Methyl-2,4-bis-phenyl-imidazol, Smp. 214-215°.

Patentansprüche

1. Verfahren zur Herstellung von Imidazolen der Formel

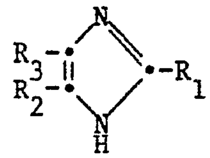

(I),

worin $R_1$ sowie mindestens einer der Reste $R_2$ und $R_3$ einen über ein Kohlenstoffatom gebundenen organischen Rest und ein davon verschiedener Rest $R_2$ oder $R_3$ Wasserstoff darstellt, wobei über gesättigte Kohlenstoffatome gebundene nicht-aromatische Reste $R_2$ und $R_3$ auch miteinander verbunden sein können, mit der Massgabe, dass $R_1$ von Resten der Formeln $-A-O-R_4$, $-A-S(O)_n-R_4$ und $-A-R_5$, in denen A einen zweiwertigen Kohlenwasserstoffrest, $R_4$ einen gegebenenfalls substituierten Kohlenwasserstoffrest, n 0, 1 oder 2 und $R_5$ gegebenenfalls verestertes oder amidiertes Carboxy bedeutet, verschieden ist, wenn $R_2$ und $R_3$ aromatische Reste darstellen, und ihrer Salze, dadurch gekennzeichnet, dass man ein reaktionsfähiges verestertes Hydroxyketon der Formel

$$R_2-CH(X)-C(=O)-R_3 \qquad (IX)$$

worin X eine reaktionsfähige veresterte Hydroxygruppe darstellt, oder ein Salz davon, mit einem Ammoniumsalz der Formel

$$R_1 - COO^{\ominus} NH_4^{\oplus} \qquad (X),$$

erforderlichenfalls in Gegenwart von Ammoniak oder eines Ammoniakliefernden Mittels, umsetzt.

2. Verfahren nach Anspruch 1, dadurch gekennzeichnet, dass man von Verbindungen der Formel IX ausgeht, worin X mit einer starken Mineralsäure oder starken organischen Sulfonsäure verestertes Hydroxy, wie mit einer Halogenwasserstoffsäure, z.B. Chlor- oder Bromwasserstoffsäure, oder einer Niederalkan- oder Arylsulfonsäure, wie Methansulfonsäure oder einer gegebenenfalls substituierten Benzolsulfonsäure, verestertes Hydroxy ist.

3. Verfahren nach einem der Ansprüche 1 und 2, dadurch gekennzeichnet, net, dass man eine Verbindung der Formel IX mit etwa äquimolaren Menge eines Ammoniumsalzes der Formel X und der mindestens äquimolekularen Menge, vorzugsweise jedoch der etwa 3- bis 5-fach molaren Menge von Ammoniak oder einem mindestens molaren Ueberschuss des Salzes der Formel X oder eines Ammoniumsalzes einer verglichen mit der Säure der Formel $R_1$-COOH (XI) schwächeren Säure, z.B. mit Ammoniumcarbonat oder Ammoniumcarbaminat, als Ammoniak-lieferndes Mittel umsetzt.

4. Verfahren nach einem der Ansprüche 1 bis 3, dadurch gekennzeichnet, dass man eine Verbindung der Formel IX mit der mindestens doppeltmolaren Menge eines Ammoniumsalzes der Formel X umsetzt.

5. Verfahren nach einem der Ansprüche 1 und 2, dadurch gekennzeichnet, dass man eine Verbindung der Formel IX worin X Halogen, wie Brom, bedeutet, bei einer Reaktionstemperatur von etwa 100°C mit einem Ammoniumsalz der Formel X im Ueberschuss, z.B. in einem Verhältnis gegenüber dem Ester der Formel IX von etwa 4:1 bis etwa 6:1, umsetzt.

6. Verfahren nach einem der Ansprüche 1 bis 5, dadurch gekennzeichnet, dass man das Ammoniumsalz der Formel X unter den Reaktionsbedingungen <u>in situ</u> bildet, indem man im Reaktionsgemisch die Säure der Formel $R_1$-COOH (XI) vorlegt und mit flüssigem oder gasförmigem Ammoniak versetzt, oder eine Säure der Formel $R_1$-COOH (XI) mit Ammoniak oder einem Ammoniumsalz einer verglichen mit der Säure der Formel $R_1$-COOH (XI) schwächeren Säure, wie Kohlensäure, z.B. mit Ammoniumcarbonat oder -carbaminat, umsetzt.

7. Verfahren nach einem der Ansprüche 1 bis 6, dadurch gekennzeichnet, dass man als Lösungsmittel die entsprechende Säure der Formel $R_1$-COOH (XI), gegebenenfalls im Ueberschuss, verwendet.

8. Verfahren nach einem der Ansprüche 1 bis 7, dadurch gekennzeichnet, dass man Imidazole der Formel I, worin $R_1$ Niederalkyl, Cycloalkyl oder gegebenenfalls durch Niederalkyl, Niederalkoxy oder Halogen substituiertes Phenyl ist, einer der Reste $R_2$ und $R_3$ gegebenenfalls durch Niederalkyl, Niederalkoxy, Niederalkylthio, Niederalkansulfonyl, Hydroxy oder Halogen substituiertes Phenyl und der andere einen gegebenenfalls N-oxidierten 6-gliedrigen heteroaromatischen Rest mit 1 oder 2 Stickstoffatomen bedeutet, oder deren Salze herstellt.

9. Verfahren nach einem der Ansprüche 1 bis 7, dadurch gekennzeichnet, dass man Imidazole der Formel I, worin $R_1$ einen verzweigten Niederalkylrest bis und mit 4 C-Atomen oder einen gegebenenfalls durch Halogen bis und mit Atomnummer 35, wie Chlor, substituierten Phenylrest darstellt, mindestens einer der Reste $R_2$ und $R_3$ einen durch Halogen bis und mit Atomnummer 35, Niederalkyl bis und mit 4 C-Atomen, Niederalkoxy mit bis und mit 4 C-Atomen oder Niederalkylthio mit bis und mit 4 C-Atomen, wie Methylthio, p-substituierten, durch Niederalkoxy mit bis und mit 4 C-Atomen, wie Methoxy, m- und p-substituierten oder durch Halogen der Atomnummer bis und mit 35, wie Chlor o- und p-substituierten Phenylrest und ein davon verschiedener Rest $R_1$ oder $R_2$ Pyridyl bzw. 1-Oxido-pyridyl, wie 3-Pyridyl, Furyl, wie 2-Furyl, oder Thienyl, wie 2-Thienyl, bedeutet, oder deren Salze herstellt.

10. Verfahren nach einem der Ansprüche 1 bis 12, dadurch gekennzeichnet, dass man eine der in einem der Beispiele 1 bis 10 genannten Verbindungen der Formel I, insbesondere 2-tert.-Butyl-4(5)-phenyl-5(4)-(3-pyridyl)-imidazol oder ein Salz davon herstellt.